# DEMANDE DE BREVET EUROPEEN

(11) **EP 1 977 697 A1**
(43) Date de publication de la demande: **08.10.2008**
(21) Numéro de dépôt: 08305080.7
(22) Date de dépôt: 02.04.2008
(51) Int. Cl.: A61B 17/02

(54) **Ecarteur sternal multifonctions**

(30) Priorité: 02.04.2007 FR 0702402
(71) Demandeur: Landanger, 52000 Chaumont (FR)
(72) Inventeur: Landanger, Benoît, 52360, Changey (FR); Desire, Pierre-Denis, 52000, Chaumont (FR)
(74) Mandataire: Oudin, Stéphane

(57) **Abrégé**

L'invention concerne un écarteur sternal multifonctions (1), pour écartement latéro-latéral ou écartement antéro-postérieur sans point d'appui ni support se trouvant hors de l'ouverture pratiquée, comportant une crémaillère dentée (2) munie d'un bras fixe (3) et d'un étrier mobile (4) se déplaçant le long de ladite crémaillère (2), lesdits bras fixe (3) et étrier mobile (4) étant chacun prolongé perpendiculairement à ladite crémaillère (2) respectivement par une première (5) et une seconde (6) tige pleine dont la section droite comporte des pans coupés et sur lesquelles peut coulisser une valve d'appui ou au moins une valve libre composées chacune d'un profilé et d'un manchon remarquable en ce que les tiges pleines (5,6) ont une section à au moins cinq pans et en ce que dans une configuration d'écartement antéro-postérieur, la première tige pleine (5) accueille une troisième (9) valve d'appui qui est sans point d'appui déporté ni support situé hors de l'ouverture pratiquée et dont le manchon (92) possède un trou débouchant assurant une liaison mécanique de type glissière avec ladite tige pleine (5) et permettant une orientation angulaire de l'axe du profilé (91) de la valve d'appui de 0 à 90° par rapport à la crémaillère (2), et la seconde tige pleine (6) accueille au moins une valve libre (10), qui est sans point d'appui déporté ni support situé hors de l'ouverture pratiquée, dont le manchon (102) possède un trou cylindrique débouchant assurant ainsi une liaison mécanique de type pivot glissant avec ladite tige pleine (6).

## Description

La présente invention concerne un écarteur sternal multifonctions permettant d'écarter le sternum soit de façon latéro-latérale, soit de façon antéro-postérieure sans point d'appui ou support se trouvant hors de l'ouverture pratiquée, et qui est destiné notamment aux interventions sur les artères mammaires.

Lors des interventions chirurgicales, un écartement latéro-latéral du sternum, parallèlement au plan frontal du patient, permet d'exposer le coeur, l'aorte, les poumons et les régions voisines.

Par contre, pour accéder facilement aux artères mammaires situées sur la face interne de part et d'autre du sternum, notamment lors d'un pontage coronarien, le chirurgien doit pratiquer un écartement antéro-postérieur du sternum, afin de soulever une partie du sternum par rapport à l'autre, suivant un plan incliné de 30 à 60° par rapport au plan sagittal du patient.

Parmi les écarteurs sternaux, on connaît déjà plusieurs types de dispositifs, par exemple : l'écarteur de DUBOST, l'écarteur de CARPENTIER ou l'écarteur de FINOCHIETTO. Tous ces dispositifs possèdent une crémaillère pour écarter progressivement deux bras munis de valves en forme générale de U, emboîtant le sternum et l'amorce des côtes, depuis une position serrée, lors de l'introduction de l'écarteur dans l'incision, jusqu'à un écartement suffisant pour accéder à la partie du patient souhaitée.

Ainsi un premier écarteur, dit écarteur de DUBOST, permettant un mouvement latéro-latéral, possède une crémaillère dentée portant deux bras servant de support à des valves. Les valves sont montées glissantes sur les bras et sont complètement interchangeables et réversibles. Toutefois, de part sa conception, la liaison glissière entre les bras et les valves ne permet pas d'orienter angulairement (notamment à 45°) les valves par rapport à l'axe longitudinal de la crémaillère, interdisant ainsi tout écartement antéro-postérieur du sternum.

Un autre type d'écarteur sternal, permettant un mouvement antéro-postérieur, comme celui décrit dans le brevet FR 2 599 238, est constitué d'une crémaillère dentée portant deux bras servant de support à des valves d'écarteur. L'un des bras porte une valve d'appui amovible dont l'axe se trouve dans un plan faisant un angle de 30 à 60° avec le plan de la crémaillère et l'autre bras est une tige ronde portant deux valves en forme de griffes fermées, amovibles et montées folles. Ce type d'écarteur, de part l'inclinaison de sa valve d'appui et la liberté de ses griffes, ne permet pas de réaliser un écartement latéro-latéral. De plus, les valves ne sont pas interchangeables.

Enfin , on connaît aussi d'autres écarteurs de type antéro-postérieur, par exemple décrits dans les brevets US 5 908 382 ou WO 99/15081, qui nécessitent, en plus, l'utilisation soit d'un point d'appui, déporté ou non, sur le patient, soit d'un support se trouvant hors du champ opératoire, par exemple de type palan. Ces écarteurs sont de ce fait particulièrement encombrants et difficiles à mettre en oeuvre.

Ces différents écarteurs connus ne sont toutefois prévus que pour une seule fonction, c'est-à-dire qu'ils peuvent soit écarter le sternum, soit le soulever, mais en aucun cas ils ne pourront être utilisés pour remplir ces deux fonctions. Ce problème implique donc, pour une intervention sur les artères mammaires, d'acquérir puis de faire entrer dans le champ opératoire deux dispositifs complets, et impose au chirurgien, après avoir écarté le sternum, de retirer entièrement l'écarteur à mouvement latéro-latéral et de mettre à la place l'écarteur à mouvement antéro-postérieur pour soulever le sternum et ainsi accéder auxdites artères.

On connaît à cet égard des écarteurs prévus pour réaliser les deux types d'écartement, après modification des valves et/ou de l'angle d'inclinaison de ces dernières.

Ainsi, le brevet américain US 4 852 552 décrit un écarteur qui est constitué d'une crémaillère dentée portant deux bras de section rectangle sur leur portion servant de support à des valves d'écarteur pouvant coulisser sur lesdits bras. Les deux bras sont munis, à leur extrémité liée à la crémaillère, d'un système de réglage et de verrouillage de leur rotation autour de leur axe, ce qui permet d'incliner les valves, montées non rotatives sur les bras, par rapport au plan défini par la crémaillère. Ce type d'écarteur est relativement encombrant du fait du dispositif de réglage. En outre, il présente un risque non négligeable de glissement ou de déboîtement hors de l'ouverture pratiquée dans le patient lorsqu'il est en configuration d'écartement antéro-postérieur puisque les deux valves sont montées fixes en rotation autour de l'axe des bras.

Par ailleurs, le modèle d'utilité DE 200 03 335 U divulgue un écarteur bimodal dont les bras ont une section rectangle. Pour éviter les risques de déboîtement, ce dispositif en configuration d'écartement antéro-postérieur met en oeuvre sur le bras fixe en plus des valves une plaque additionnelle d'appui sur la partie extérieure des côtes du patient, afin de générer un contre-appui au mouvement d'écartement. En outre, la valve montée sur le bras mobile crée un point d'appui déporté, est montée sur un pivot et est munie d'un dispositif de réglage de son inclinaison autour de l'axe du bras, au moyen d'une vis en butée. Ce dispositif recourt à des points d'appui externes situés de part et d'autre de l'ouverture pratiquée, ce qui est plutôt traumatisant pour le patient car cela engendre des hématomes importants et des risques de luxation élevés, notamment sur la bordure costale tenue par la valve montée sur le bras mobile.

La présente invention a pour but de remédier à ces divers inconvénients en proposant un écarteur sternal multifonctions, muni de différentes valves adaptées, interchangeables et réversibles, permettant d'écarter le sternum soit de façon latéro-latérale, soit de façon antéro-postérieure sans point d'appui déporté ou support se trouvant hors de l'ouverture pratiquée. De plus, dans la mesure où le chirurgien se sert de plusieurs éléments communs du dispositif pour effectuer des actes différents et où seules les valves sont changées pour passer d'une configuration à l'autre, cette invention permet une réduction de coût et de temps, grâce notamment à la stérilisation et à la manipulation d'un seul ensemble crémaillère-bras mobile-manivelle.

A cet égard, la présente invention a pour objet un écarteur sternal multifonctions comportant une crémaillère dentée munie d'un bras fixe formant talon et d'un étrier mobile se déplaçant le long de l'axe longitudinal de ladite crémaillère, lesdits bras fixe et étrier mobile étant chacun prolongé perpendiculairement à ladite crémaillère respectivement par une première et une seconde tige pleine dont la section droite comporte des pans coupés et sur lesquelles peut coulisser une valve d'appui ou au moins une valve libre composées chacune d'un profilé et d'un manchon.

Cet écarteur est remarquable en ce que les tiges pleines ont une section à au moins cinq pans et en ce que dans une configuration d'écartement latéro-latéral, la première tige pleine accueille une première ou une quatrième valve d'appui et la seconde tige accueille respectivement une seconde ou une cinquième valve d'appui, les manchons respectifs desdites valves possédant un trou débouchant présentant une section droite de même configuration que celle respectivement de la première ou de la deuxième tige pleine, assurant ainsi une liaison mécanique de type glissière entre lesdites valves d'appui et lesdites tiges pleines, tandis que dans une configuration d'écartement antéro-postérieur, la première tige pleine accueille une troisième valve d'appui qui est sans point d'appui déporté ni support situé hors de l'ouverture pratiquée et dont le manchon possède un trou débouchant présentant une section droite de même configuration que celle de la première tige pleine, assurant ainsi une liaison mécanique de type glissière entre ladite valve d'appui et ladite tige pleine et permettant une orientation angulaire de l'axe du profilé de la valve d'appui de 0 à 90° par rapport à l'axe longitudinal de la crémaillère, et la seconde tige pleine accueille au moins une valve libre, qui est sans point d'appui déporté ni support situé hors de l'ouverture pratiquée, dont le manchon possède un trou cylindrique débouchant qui a un périmètre circonscrit au contour de la section droite de la seconde tige pleine, assurant ainsi une liaison mécanique de type pivot glissant entre ladite valve libre et ladite tige pleine.

Le terme liaison mécanique désigne ici une mise en relation de deux pièces par contact physique permettant de les rendre partiellement ou totalement solidaires (en supprimant ou non l'un des six degrés de liberté : 3 translations, 3 rotations, d'une pièce par rapport à l'autre) et en contribuant à la transmission éventuelle d'un effort entre les deux pièces.

Ainsi une liaison mécanique de type glissière est telle que le seul degré de liberté autorisé entre les pièces est un mouvement de translation rectiligne.

S'agissant de la liaison mécanique de type pivot glissant, l'une des pièces peut avoir soit un mouvement de translation rectiligne suivant un axe, soit un mouvement de rotation autour du même axe par rapport à l'autre pièce.

Selon une autre caractéristique de l'invention, la section droite des tiges pleines portées par le bras fixe et l'étrier mobile est avantageusement un octogone régulier. En effet, cette section octogonale a pour avantage d'une part d'avoir une meilleure résistance mécanique que celle du carré inscrit dans le même cercle et d'autre part de permettre d'orienter angulairement les différentes valves d'appui (car certains chirurgiens souhaitent faire pivoter lesdites valves de 45°, notamment lors de l'écartement antéro-postérieur).

Il va de soi que pour la section des tiges pleines, on peut envisager soit d'autres sections polygonales régulières à au moins cinq pans telles que par exemple un un dodécagone, soit un cercle comportant des pans coupés, sans sortir du cadre de la présente invention.

Selon une variante particulièrement avantageuse de l'invention, les deux tiges pleines et les valves d'appui sont respectivement identiques entre elles.

Afin de faire mieux ressortir d'autres avantages et caractéristiques de la présente invention, on décrira ci-après à titre d'exemple non limitatif, une forme d'exécution préférée de ladite invention, en référence aux dessins annexés sur lesquels :
- La figure 1 est une vue en perspective de l'écarteur sternal multifonctions sans valve,
- La figure 2 est une vue en perspective de l'écarteur sternal multifonctions en configuration permettant un écartement latéro-latéral,
- La figure 3 est une vue en perspective de l'écarteur sternal multifonctions en configuration permettant un écartement antéro-postérieur,
- La figure 4 est un détail de la figure 3,
- La figure 5 est une vue en perspective de l'écarteur sternal multifonctions en configuration pour valve malléable avec deux bras supplémentaires.

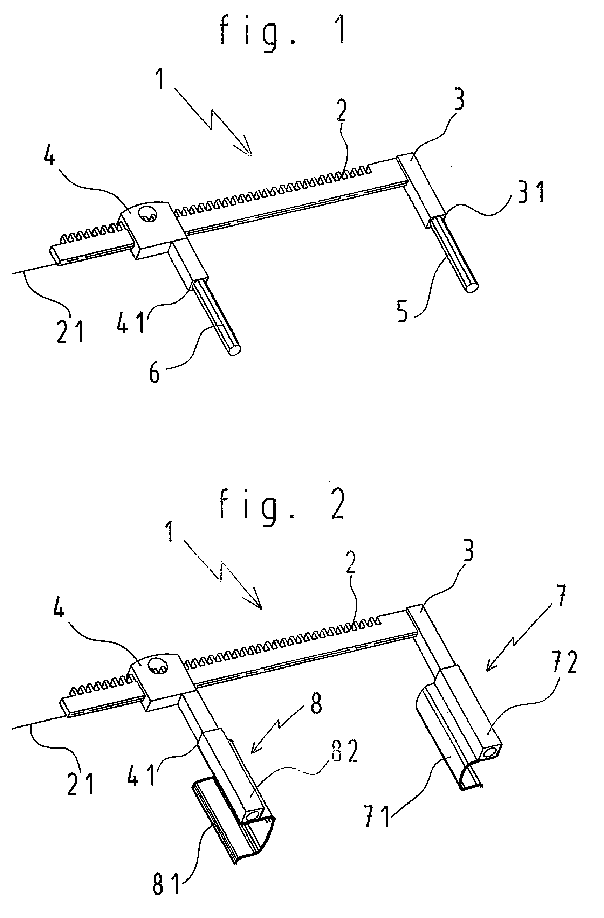

En référence aux figures 1 et 2, l'écarteur sternal orientable 1 selon l'invention est monté suivant une première configuration permettant un écartement latéro-latéral du sternum. L'écarteur 1 comprend une crémaillère 2 et un bras fixe 3 formant talon, issu d'une extrémité de ladite crémaillère 2, perpendiculairement à son axe longitudinal 21. Le bras fixe 3 se prolonge, du coté opposé à la denture de la crémaillère 2, perpendiculairement à sa face supérieure 31 par une première tige pleine 5 rectiligne dont la section droite est un octogone régulier dont deux faces opposées sont perpendiculaires à l'axe longitudinal 21 de ladite crémaillère 2. Le long de cette première tige pleine 5 coulisse librement sans rotation la première valve d'appui 7. Ladite valve d'appui 7 se compose d'un profilé 71 en forme générale de U à ailes égales et d'un manchon 72 formant rebord issu perpendiculairement de l'extrémité libre d'une des ailes dudit profilé 71, vers l'extérieur dudit profilé 71.

Afin de permettre le coulissement sans rotation de la première valve d'appui 7 le long de la première tige pleine 5, le manchon 72 possède le long de son axe longitudinal un orifice débouchant dont la section droite est octogonale et homothétique à celle de ladite tige pleine 5. Il va de soi que la section de l'orifice du manchon 72 doit être homothétique et légèrement supérieure à celle de ladite tige pleine 5 pour garantir un coulissement sans rotation.

Enfin, pour permettre une orientation de la première valve d'appui 7 parallèle au plan longitudinal de la crémaillère 2, on s'assure que deux faces opposées de l'orifice octogonal du manchon 72 soient parallèles aux ailes du profilé 71. La première valve d'appui 7 coulisse donc le long de la première tige pleine 5 perpendiculairement à l'axe longitudinal 21 de la crémaillère 2 et de telle sorte que les ailes du profilé 71 s'étendent du coté opposé à un étrier mobile 4, engagé le long de la crémaillère 2.

L'étrier mobile 4, de préférence parallélépipédique, est mu par un dispositif de rouleaux entraînés par une manivelle (non représenté sur les figures). Ce dispositif d'entraînement est semblable à ceux des écarteurs existants et ne sera pas décrit plus en détail, car il est bien connu de l'homme du métier, et ne fait partie de l'invention. L'étrier mobile 4 se prolonge, du coté opposé à la denture de la crémaillère 2, perpendiculairement à sa face supérieure 41, par une seconde tige pleine 6 rectiligne. La section droite de ladite tige pleine 6 est avantageusement un octogone régulier dont deux faces opposées sont perpendiculaires à l'axe longitudinal 21 de ladite crémaillère 2. Ainsi lorsque l'étrier 4 est engagé sur la crémaillère 2, la seconde tige pleine 6 se trouve dans le plan longitudinal de ladite crémaillère 2 et est parallèle à la première tige pleine 5. Le long de cette seconde tige pleine 6 coulisse librement sans rotation une seconde valve d'appui 8, semblable à la valve d'appui 7 décrite précédemment, c'est-à-dire comportant un profilé 81 en forme générale de U à ailes égales et d'un manchon 82 formant rebord. La seconde valve d'appui 8 est montée de telle sorte que les ailes respectives des profilés 71 et 81 soient parallèles et situées du même coté par rapport au plan longitudinal de la crémaillère 2, et que les ailes du profilé 81 s'étendent du coté opposé aux ailes du profilé 71.

Afin d'avoir des valves d'appui 7 et 8 strictement identiques et donc un écarteur 1 économiquement avantageux, il va de soi que les sections respectives des tiges pleines 5 et 6 sont de préférence identiques. Les ailes respectives des profilés 71 et 81 sont alors sensiblement coplanaires. Grâce à leur configuration particulière, les profilés 71 et 81 se trouvent désaxés par rapport à l'axe longitudinal de la crémaillère 2 du coté opposé à la manivelle, facilitant ainsi l'utilisation de l'écarteur 1, car la crémaillère 2 se trouve en dehors du patient lors d'un écartement latéro-latéral du sternum.

L'écarteur 1, monté suivant la première configuration permettant un écartement latéro-latéral telle que précédemment décrite en référence aux figures 1 et 2, s'utilise de la façon suivante : le chirurgien incise le sternum, introduit l'écarteur 1 dans la découpe, après avoir pris soin de mettre en contact les âmes des profilés 71 et 81 en rapprochant à l'aide de la manivelle l'étrier mobile 4 du bras fixe 3 ; puis, à l'aide de la manivelle, il éloigne progressivement les valves d'appui 7 et 8 montées respectivement sur le bras fixe 3 et l'étrier mobile 4, et de ce fait il écarte le sternum. L'écartement du sternum se fait le long du plan longitudinal de la crémaillère, parallèlement au plan frontal du patient, permettant ainsi au chirurgien d'accéder facilement au coeur, à l'aorte, aux poumons et aux régions voisines. Compte tenu de la conception de l'écarteur sternal 1, il va de soi que le montage est réversible, c'est-à-dire que la crémaillère peut-être placée indifféremment soit vers le haut ou vers le bas du patient, soit vers la gauche ou vers la droite du patient.

On décrit maintenant, en référence aux figures 1, 3 et 4, l'écarteur 1, selon l'invention, monté suivant une seconde configuration permettant un écartement antéro-postérieur sans point d'appui déporté ou support se trouvant hors de l'ouverture pratiquée. Ainsi, la première 5 et la seconde 6 tiges pleines accueillent respectivement une troisième valve d'appui 9 et au moins une valve libre 10.

Cette troisième valve d'appui 9 se compose d'un profilé 91 en forme générale de U à ailes inégales et d'au moins un manchon 92 formant talon issu perpendiculairement du milieu de l'âme du profilé 91, vers l'extérieur dudit profilé 91. Le positionnement de cette troisième valve 9 sur la première tige 5 est réalisé de manière à ce que la grande aile du profilé 91 se retrouve à l'extérieur de l'ouverture (du même coté par rapport au plan longitudinal de la crémaillère 2 que la manivelle de l'étrier mobile 4) et assure ainsi un contre-appui.

Afin de permettre le coulissement sans rotation de la troisième valve d'appui 9 le long de la tige pleine 5, le manchon 92 possède le long de son axe longitudinal un orifice débouchant dont la section droite est octogonale et homothétique à celle de ladite tige pleine 5. Il va de soi que la section de l'orifice du manchon 92 doit être homothétique et légèrement supérieure à celle de ladite tige pleine 5 pour garantir un coulissement sans rotation. De plus, pour permettre une orientation de la troisième valve d'appui 9 telle que l'axe de symétrie 93 du profilé 91 forme un angle de 45° avec l'axe longitudinal 21 de la crémaillère, on s'assure que deux faces opposées de l'orifice octogonal du manchon 92 soient parallèles aux ailes du profilé 91.

La valve libre 10 se compose d'un profilé 101 en forme générale de U à ailes inégales et d'un manchon 102 de préférence cylindrique formant rebord issu perpendiculairement de l'extrémité libre de la grande aile dudit profilé 101, vers l'intérieur dudit profilé 101. Cette valve est également sans point d'appui déporté ni support situé hors de l'ouverture pratiquée.

Afin de permettre le coulissement avec rotation de la valve libre 10 le long de la seconde tige pleine 6, le manchon 102 possède le long de son axe longitudinal un trou cylindrique débouchant dont le périmètre est circonscrit au contour de la section droite de la seconde tige pleine 6. Enfin la valve 10 doit être montée sur la seconde tige pleine 6 de telle sorte que la grande aile dudit profilé 101 soit du coté de la première tige pleine 5 lorsque les profilés 91 et 101 sont situés du même coté par rapport au plan longitudinal de la crémaillère 2.

L'écarteur 1, monté suivant la seconde configuration permettant un écartement antéro-postérieur sans point d'appui déporté ni support se trouvant hors de l'ouverture pratiquée, telle que précédemment décrite en référence aux figures 1, 3 et 4 s'utilise de la façon suivante : le chirurgien introduit l'écarteur 1 dans la découpe, après avoir pris soin de rapprocher au maximum, à l'aide de la manivelle, l'étrier mobile 4 du bras fixe 3, puis emboîte la troisième valve d'appui 9 sur la partie du sternum opposée au coté du prélèvement mammaire et la valve libre 10 sur la partie du coté du prélèvement. Enfin, à l'aide de la manivelle, il écarte progressivement lesdites valves 9 et 10 montées respectivement sur le bras fixe 3 et l'étrier mobile 4. Compte tenu de l'inclinaison à 45° de la troisième valve d'appui 9, l'écartement du sternum se fait suivant un plan incliné de 30 à 60° par rapport au plan sagittal du patient, permettant au chirurgien de soulever la partie appropriée du sternum par rapport à la partie maintenue par la troisième valve d'appui 9 et ainsi d'accéder facilement à l'artère mammaire du patient.

Si le chirurgien doit intervenir sur l'artère mammaire placée de l'autre coté du patient, il retire l'étrier mobile 4, le fait pivoter de 180° puis l'engage à nouveau sur ladite crémaillère 2, enlève la valve libre 10 de la tige pleine 6 et la ré-enfile dans l'autre sens, enlève la valve d'appui 9 de la tige pleine 5 et la ré-enfile dans l'autre sens, et enfin fait pivoter de 180° l'écarteur ainsi configuré de telle sorte que le bras fixe 3 se trouve sur le coté opposé à l'artère mammaire à exposer.

On décrit enfin, en référence aux figures 1, 5 à 7, l'écarteur 1 selon l'invention monté suivant une troisième configuration permettant de réaliser un écartement latéro-latéral et d'utiliser une ou plusieurs valves malléables. Ainsi la première 5 et la seconde 6 tiges pleines accueillent respectivement une quatrième 11 et cinquième 12 valves d'appui.

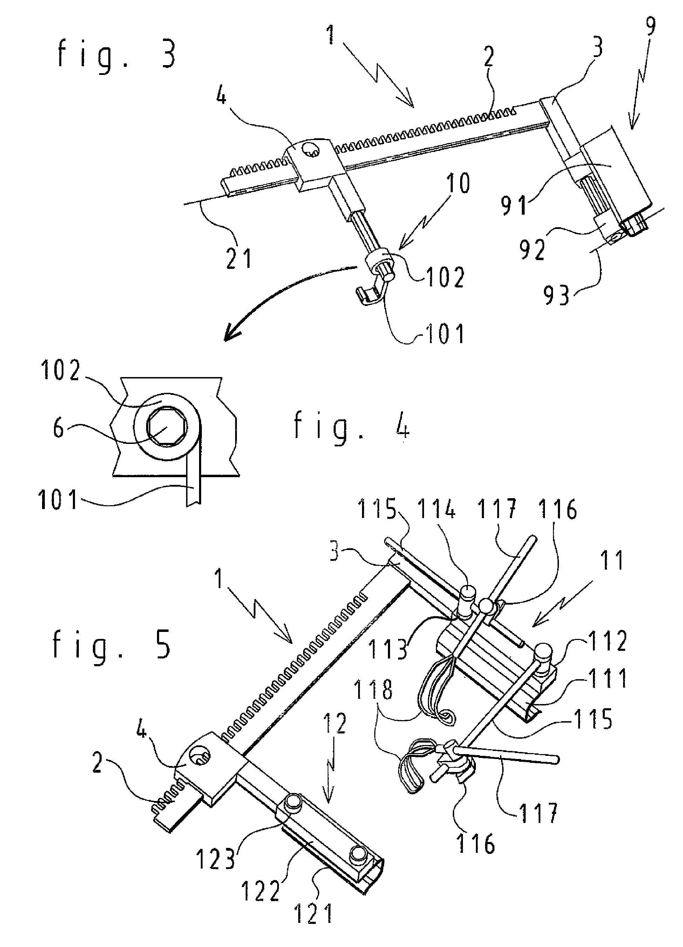

Ces quatrième 11 et cinquième 12 valves d'appui sont semblables aux première 7 et seconde 8 valves d'appui, et se composent donc respectivement d'un profilé 111 et 121 en forme générale de U à ailes égales et d'un manchon 112 et 122 formant talon. Les manchons et profilés desdites valves d'appui 11 et 12 ont les mêmes propriétés que ceux desdites valves d'appui 7 et 8. En outre, la quatrième 11 et la cinquième 12 valves d'appui possèdent, sur la face de leur manchon opposée à leur profilé, au moins deux moyens de fixation, par exemple un écrou, respectivement 113 et 123. Selon la configuration définie par la figure 5, sur les moyens de fixation 113, on peut fixer perpendiculairement au manchon 112, par exemple grâce à une vis moletée, une entretoise 114 permettant de faire coulisser, d'orienter et de fixer, perpendiculairement à ladite entretoise 114, un premier bras 115. Ce premier bras 115 permet la fixation, par l'intermédiaire d'une articulation 116, d'une valve malléable composée d'un second bras 117 et d'un crochet 118. L'articulation 116 permet de faire coulisser et tourner la valve malléable par rapport au premier bras 115.

Selon les besoins, les moyens de fixations 123 peuvent accueillir des éléments semblables à ceux décrits précédemment.

L'écarteur 1, monté suivant la troisième configuration permettant de réaliser un écartement latéro-latéral et d'utiliser une ou plusieurs valves malléables telle que précédemment décrite en référence aux figures 1 et 5, s'utilise de la façon suivante : le chirurgien incise le sternum, introduit l'écarteur 1 dans la découpe, après avoir pris soin de mettre en contact les âmes des profilés 111 et 121 en rapprochant à l'aide de la manivelle l'étrier mobile 4 du bras fixe 3 ; puis, à l'aide de la manivelle, il éloigne progressivement les valves d'appui 111 et 121 respectivement montées sur le bras fixe 3 et l'étrier mobile 4, et de ce fait il écarte le sternum. L'écartement du sternum se fait le long du plan longitudinal de la crémaillère, parallèlement au plan frontal du patient. Une fois l'écartement souhaité obtenu, le chirurgien met un place la ou les valves malléables pour maintenir écartés, sans les altérer, des organes situés plus à l'intérieur du patient, et ainsi accéder à d'autres organes placés en dessous.

Enfin, il va de soi que l'on peut passer indifféremment d'une configuration à l'autre.

Compte tenu des efforts mécaniques mis en oeuvre et du contexte sanitaire, l'homme du métier n'éprouvera aucune difficulté à choisir les matériaux adaptés pour réaliser l'écarteur sternal orientable 1 selon l'invention. On utilisera par exemple de l'acier inoxydable traité.

De même, l'homme du métier pourra facilement déterminer pour les ailes des profilés 71, 81, 91, 101, 111 et 121 une forme dépourvue d'angles vifs et tranchants et des dimensions appropriées afin d'assurer un appui convenable sur le patient, sans altérer les organes sous-jacents et minimisant l'importance des hématomes et du risque de luxation lors de l'écartement du sternum.

Enfin, il va bien entendu de soi que la présente invention n'est pas limitée aux exemples de réalisation préférentiels décrits, mais qu'elle peut être modifiée ou adaptée en fonction des besoins ou des exigences particulières, sans pour autant sortir du cadre de l'invention.

## Revendications

**1.** Ecarteur sternal multifonctions (1) permettant de réaliser soit un écartement latéro-latéral soit un écartement antéro-postérieur dans une ouverture thoracique pratiquée sur un patient, comportant une crémaillère dentée (2) munie d'un bras fixe (3) formant talon et d'un étrier mobile (4) se déplaçant le long de l'axe longitudinal (21) de ladite crémaillère (2), lesdits bras fixe (3) et étrier mobile (4) étant chacun prolongé perpendiculairement à ladite crémaillère (2) respectivement par une première (5) et une seconde (6) tige pleine dont la section droite comporte des pans coupés et sur lesquelles peut coulisser une valve d'appui (7,8,9,11,12) ou au moins une valve libre (10) composées chacune d'un profilé (71,81,91,101,111,121) et d'un manchon (72,82,92,102,112,122) **caractérisé en ce que** les tiges pleines (5,6) ont une section à au moins cinq pans et **en ce que** dans une configuration d'écartement latéro-latéral, la première tige pleine (5) accueille une première (7) ou une quatrième (11) valve d'appui et la seconde tige (6) accueille respectivement une seconde (8) ou une cinquième (12) valve d'appui, les manchons respectifs (72,82,112,122) desdites valves (7,8,11,12) possédant un trou débouchant présentant une section droite de même configuration que celle respectivement de la première (5) ou de la deuxième (6) tige pleine, assurant ainsi une liaison mécanique de type glissière entre lesdites valves d'appui (7,8,11,12) et lesdites tiges pleines (5,6), tandis que dans une configuration d'écartement antéro-postérieur, la première tige pleine (5) accueille une troisième (9) valve d'appui qui est sans point d'appui déporté ni support situé hors de l'ouverture pratiquée et dont le manchon (92) possède un trou débouchant présentant une section droite de même configuration que celle de la première tige pleine (5), assurant ainsi une liaison mécanique de type glissière entre ladite valve d'appui (9) et ladite tige pleine (5) et permettant une orientation angulaire de l'axe du profilé (91) de la valve d'appui de 0 à 90° par rapport à l'axe longitudinal (21) de la crémaillère (2), et la seconde tige pleine (6) accueille au moins une valve libre (10), qui est sans point d'appui déporté ni support situé hors de l'ouverture pratiquée, dont le manchon (102) possède un trou cylindrique débouchant qui a un périmètre circonscrit au contour de la section droite de la seconde tige pleine (6), assurant ainsi une liaison mécanique de type pivot glissant entre ladite valve libre (10) et ladite tige pleine (6).

**2.** Ecarteur sternal multifonctions (1) suivant la revendication 1, **caractérisé en ce que** la première (5) et la seconde (6) tiges pleines ont une section droite octogonale.

**3.** Ecarteur sternal multifonctions (1) suivant l'une quelconque des revendications 1 ou 2, **caractérisé en ce que** les sections droites de la première (5) et de la seconde (6) tiges pleines sont orientées de telle sorte que deux faces opposées soient perpendiculaires à l'axe longitudinal (21) de la crémaillère (2).

**4.** Ecarteur sternal multifonctions (1) suivant l'une quelconque des revendications 1 à 3, **caractérisé en ce que** la première (5) et la seconde (6) tiges pleines sont identiques.

**5.** Ecarteur sternal multifonctions (1) suivant l'une quelconque des revendications 1 à 4, **caractérisé en ce que** la première (7), la seconde (8), la quatrième (11) et la cinquième (12) valve d'appui se composent respectivement d'un profilé (71, 81, 111, 121) en forme générale de U à ailes égales et d'un manchon (72, 82, 112, 122) formant rebord issu perpendiculairement de l'extrémité libre d'une des ailes dudit profilé (71, 81, 111, 121), vers l'extérieur dudit profilé (71, 81, 111, 121).

**6.** Ecarteur sternal multifonctions (1) suivant la revendication précédente, **caractérisé en ce que** la première (7) et la seconde (8) valves d'appui sont identiques.

**7.** Ecarteur sternal multifonctions (1) suivant l'une quelconque des revendications 1 à 6, **caractérisé en ce que** la troisième valve d'appui (9) se compose d'un profilé (91) en forme générale de U à ailes inégales et d'un manchon (92) formant talon issu perpendiculairement du milieu de l'âme du profilé (91), vers l'extérieur dudit profilé (91).

**8.** Ecarteur sternal multifonctions (1) suivant l'une quelconque des revendications précédentes, **caractérisé en ce que** la valve libre (10) se compose d'un profilé (101) en forme générale de U à ailes inégales et d'un manchon (102) formant rebord issu perpendiculairement de l'extrémité libre d'une des ailes dudit profilé (101), vers l'intérieur dudit profilé (101).

**9.** Ecarteur sternal multifonctions (1) suivant l'une quelconque des revendications 1 à 6, **caractérisé en ce que** les manchons respectifs (112) et (122) de la quatrième (11) et la cinquième (12) valves d'appui comportent des moyens de fixation (113, 123) permettant de fixer par l'intermédiaire d'ensembles, composés d'une entretoise (114), d'un premier bras (115) et d'une articulation (116), des valves malléables constituées d'un second bras (117) et d'une griffe (118).

**10.** Ecarteur sternal multifonctions (1) suivant la revendication précédente, **caractérisé en ce que** la quatrième (11) et la cinquième (12) valves d'appui sont identiques.

**11.** Ecarteur sternal multifonctions (1) suivant l'une quelconque des revendications 1, 5 ou 6, **caractérisé en ce que** la première (7) et la seconde (8) valves d'appui sont orientées de manière à être symétriques par rapport à un plan perpendiculaire à l'axe longitudinal (21) de ladite crémaillère (2) et à ce que les ailes de leurs profilés respectifs (71, 81) sont parallèles et non en regard, et **en ce que** la première (7) et la seconde (8) valves d'appui sont respectivement montées sur la première tige pleine (5) et la seconde tige pleine (6), de sorte à constituer un dispositif permettant un écartement latéro-latéral du sternum.

**12.** Ecarteur sternal multifonctions (1) suivant les revendications 1, 7 ou 8, **caractérisé en ce que** la troisième valve d'appui (9) est orientée de telle sorte que le plan de symétrie (93) du profilé (91) forme un angle de 45° avec l'axe longitudinal (21) de la crémaillère (2) et que les ailes du profilé (91) s'étendent du coté opposé à l'étrier mobile (4), et **en ce que** la première valve d'appui (9) et au moins une quatrième valve libre (10) sont respectivement montées sur la première tige pleine (5) et la seconde tige pleine (6), de sorte à constituer un dispositif permettant un écartement antéro-postérieur du sternum sans point d'appui ou support se trouvant hors de l'ouverture pratiquée.

**13.** Ecarteur sternal multifonctions (1) suivant l'une quelconque des revendications 1, 9 ou 10, **caractérisé en ce que** la quatrième valve d'appui (11) et la cinquième valve d'appui (12) sont orientées de manière à être symétriques par rapport à un plan perpendiculaire à l'axe longitudinal (21) de ladite crémaillère (2) et à avoir les ailes de leurs profilés respectifs (111, 121) parallèles et non en regard, et **en ce que** la quatrième valve d'appui (11) et la cinquième valve d'appui (12) sont respectivement montées sur la première tige pleine (5) et la seconde tige pleine (6) de sorte à constituer un dispositif permettant un écartement latéro-latéral du sternum et l'utilisation de valves malléables.

**14.** Ecarteur sternal multifonctions (1) suivant l'une quelconque des revendications précédentes, **caractérisé en ce que** son montage est réversible de sorte que la crémaillère peut être placée indifféremment soit vers le haut ou vers le bas du patient, soit vers la gauche ou vers la droite du patient.
